# EUROPEAN PATENT SPECIFICATION

(11) **EP 4 346 559 B1**
(45) Date of publication and mention of the grant of the patent: **06.08.2025**
(21) Application number: 22733322.6
(22) Date of filing: 01.06.2022
(51) Int. Cl.: A61M 37/00, A61B 5/145, A61B 10/00, A61B 5/00

(54) **SKIN PENETRATION DEVICE AND SKIN-PIERCING COMPONENT**
HAUTPENETRATIONSVORRICHTUNG UND HAUTDURCHDRINGENDE KOMPONENTE
DISPOSITIF DE PÉNÉTRATION CUTANÉE ET COMPOSANT DE PERFORATION CUTANÉE

(30) Priority: 01.06.2021 GB 202107795
(43) Date of publication of application: 10.04.2024
(73) Proprietor: MEDIDESIGN LIMITED, Holywood Down BT18 9DP (GB)
(72) Inventor: BAMBRICK, Michael, Holywood Down BT18 9DP (GB); MCGILLOWAY, Stephen, Belfast Antrim BT1 6PJ (GB); MAGOWAN, Matt, Belfast Antrim BT1 6PJ (GB)
(74) Representative: Hanna, John Philip
(86) International application number: PCT/EP2022/064900
(87) International publication number: WO 2022/253893

(56) References cited:
- WO-A1-2018/206825
- WO-A2-2011/053019
- US-A- 5 097 810
- US-A1- 2008 269 635
- US-A1- 2011 105 951

## Description

The present invention relates to a skin penetration device and in particular to a skin penetration device that can contain a substance to be applied to the skin. The present invention further relates to a skin-piercing component of a skin penetration device.

The practice of determining allergies in an individual by pricking the skin and introducing a test substance to the pricked skin to elicit an allergic reaction is well known and widely used. In its simplest form, the test may be performed by placing a drop of an allergenic substance in solution on the skin and then using a needle or lancet to penetrate the skin to allow the substance to enter the skin. If the recipient is allergic to the substance, then the skin where the allergen was introduced will redden and may develop into an itchy bump. Such tests require only minimal preparation, are quick to administer, and are suitable for preliminary tests or when it is desired to test a single potentially allergenic substance. However, the test lacks consistency because the quantity of the allergenic substance being administered, and the depth and width of the prick are determined by the person administering the test and may vary considerably between tests. If this test were used to compare a variety of substances and/or a range of concentrations on an individual, the accuracy and reliability of the results would be questionable. Further, there is always the risk that the person administering the test penetrates the skin by more than is necessary, thereby causing undue pain and discomfort to the recipient, especially if they are indeed allergic to the substance that is introduced into the skin. There is therefore a need to control both the quantity and/or concentration of the substance to be introduced and the depth of penetration of needle or lancet into the skin.

Efforts have been made to improve the reliability and efficiency of skin prick tests. Multiple lancet tests are known which involve an array of needles arranged spaced apart on a plastic frame that can be pressed into the skin to administer multiple tests simultaneously. Initially, the person administering the test sets up an array of wells containing the test substances at the desired concentrations. The array of needles is then dipped into the array of wells to coat the needles with the substance, and then the needles are applied to the recipient's skin. This improves the efficiency of administering the test, but it still takes some time to prepare the wells, and it is difficult to apply an equal force to all the needles when pressing the frame onto the recipient's skin. Further, the amount of substance on the needle may vary depending on the quantity and concentration of the substance in each well, and on how evenly the needles were submerged in the wells prior to administering the test. US2008269635 describes a minimally invasive skin testing system with a microneedle array, encapsulated allergens and an activation system. US2003181936 describes a microneedle device with micro projections folded from the substrate having penetration depth control.

Accordingly, further improvements to skin prick tests are required and the present invention obviates or mitigates the problems outlined above.

The invention is defined in independent claim 1. Further aspects are defined in dependent claims 2-15. The methods of manufacturing and of use mentioned as such herein are not part of the claimed invention and are left for illustrative purposes.

According to a first aspect of the invention, there is provided a skin-piercing component of a skin penetration device as defined in independent claim 1 and subsequent dependent claims 2-5.

According to a second aspect of the invention there is provided a skin penetration device for administering a test substance to the skin of a recipient, the skin penetration device comprising:
- a first layer,
- a second layer disposed on the first layer, the second layer comprising at least one protrusion thereby providing a hollow space within the protrusion and between the first and second layers for containing a substance to be applied to the skin of the recipient,
- a third layer disposed on the second layer, the third layer comprising an aperture and being arranged such that the protrusion of the second layer is located within the aperture,
- a fourth layer disposed on the third layer, covering the aperture of the third layer, and
- a skin-piercing component as claimed in independent claim 1 and dependent claims 2-5 disposed within and/or above the aperture of the third layer and between the second and fourth layers.

The skin penetration device is suitable for administering an allergenic substance to a recipient. Advantageously, the quantity and concentration of the substance can be predetermined by the manufacturer thereby improving the reliability and accuracy of the test results.

In one embodiment, the first layer is placed on the skin of a recipient in use, but in other embodiments a further layer or substance may be present between the first layer and the skin of the recipient.

Ideally, the second layer comprises a plurality of protrusions, the third layer comprises a plurality of corresponding apertures, and there is a corresponding plurality of skin-piercing components. Advantageously, a single skin penetration device may be used to administer a plurality of substances, or the same substance at different concentrations, to the recipient.

Ideally, the skin-piercing component is adhered to or integrally formed with the fourth layer. This retains the skin-piercing component in a position that is spaced apart from the protrusion of the second layer, thereby preventing the skin-piercing component from unintentionally penetrating the second layer, for example, during transportation of the skin penetration device.

Preferably, the thickness of the third layer functions to space the fourth layer, and therefore the skin-piercing component, apart from the protrusion of the second layer. This again prevents the skin-piercing component from unintentionally penetrating the second layer. The thickness of the third layer also provides the skin penetration device with improved structural integrity and rigidity, thereby making it even less likely that the device may be damaged, for example, during transport.

Ideally, the third layer has a thickness of between 1 and 10 mm or more preferably between 1.5 and 5 mm. Preferably, the third layer is formed of a rigid and/or non-compressible and/or non-flexible plastic. In another embodiment, the third layer is formed from a flexible and/or compressible material, most preferably, a flexible and/or compressible plastic material.

Ideally, the protrusion of the second layer is a domed or generally dome-shaped protrusion. The protrusion may be referred to as a "blister".

The skin-piercing component has a base. One or more piercing elements extend from the base. When assembled, the base is oriented such that it is facing or is abutting the fourth layer, with the one or more piercing elements extending towards the protrusion of the second layer. Advantageously, this configuration also limits the depth of penetration of the piercing element because the base prevents the piercing element from being inserted any further when the base abuts the second and first layers.

Preferably, the skin-piercing component is deformable.

The skin-piercing component has a rest configuration and is configured to be biased towards the rest configuration such that it may be deformed but returns to the rest configuration when the deforming force is removed. Advantageously, if the skin-piercing component is deformed when it is pressed into the recipient's skin, on removal of the deforming force, the skin-piercing component will revert to the rest configuration. When doing so, this can draw the piercing element out of the skin, thereby preventing the piercing element from remaining in the recipient's skin for any longer than necessary and preventing the need to manually urge the piercing element out of the skin.

Ideally, the skin-piercing component is configured to be deformed when it is pressed against a substantially flat surface, such as the skin of a recipient.

Preferably, the piercing element is located substantially centrally on the base of the skin-piercing component.

Ideally, part of the piercing element is pointed or sharpened. Preferably, the piercing element has a pointed or sharpened end and is connected to the base of the skin-piercing component at the other end opposing the pointed or sharpened end.

Ideally, the base has an inner portion and an outer portion. Ideally, the piercing element is connected to the base at the inner portion.

Preferably, at least part of the outer portion is deflected towards the pointed or sharpened part or end of the piercing element, and/or towards the second layer when the skin penetration device is assembled.

Ideally, the base slopes downwardly away from where it is connected to the piercing element, the slope extending in a direction towards the pointed or sharpened part/end of the piercing element, or towards the axis of the piercing element or towards the second layer.

Preferably, the base is substantially dome shaped. Ideally, the base is a hollow dome. Ideally, the piercing element is arranged extending from the top of the dome in a direction towards or past the bottom of the dome. When in use, the base is pressed towards the skin of the recipient and the piercing element is inserted into the skin. The base is then flattened and may be converted into a flattened or inverted configuration. This generates potential energy as the skin-piercing component is biased towards the rest configuration. When the force pressing the skin-piercing component into the skin is removed, the base of the skin-piercing component reverts to the rest configuration thereby drawing the piercing element out of the skin. The skin-piercing component is therefore self-retracting.

Ideally, the domed shape of the base is less than a hemisphere and may be similar to a "saucer dome" in shape.

Preferably, the base of the skin-piercing component is configured to enable the base to move between the dome shape in the rest configuration to a flattened or inverted configuration. Ideally, the base of the skin-piercing component has one or more incisions or cut-outs to enable it to be flattened. The skilled person will understand that the incisions or cut-outs need not be formed by physically cutting the base but may instead be formed as part of the design of the base during manufacture. In one embodiment, the base has an outer portion formed of a plurality of flanges extending out from the inner portion. The spacing between the flanges enables the base to be moved between the flattened and rest configurations.

The piercing element is formed by punching out a part of the base of the skin-piercing component and bending the punched-out part away from the base. This simplifies the manufacturing process because it is not necessary to join a separate piercing element to the base to form the skin-piercing component.

Ideally, the piercing element is generally triangular in shape.

Preferably, the skin-piercing component is formed from spring steel.

Preferably, the skin-piercing component is configured to provide tactile feedback to the user when sufficient force has been applied to the component.

Ideally, the skin-piercing component is arranged to spring from the rest configuration to a flattened or inverted configuration when a sufficient force has been applied. This sudden change in configuration when the skin-piercing component is pressed towards the skin provides the person administering the device with tactile feedback and assures the person that sufficient force has been applied and the skin-piercing component has pierced the skin. This avoids unnecessary discomfort to the recipient because the person operating the skin penetration device can immediately stop forcing the skin-piercing component towards the skin when the tactile feedback is received. As the skin-piercing component is biased towards the rest configuration, it will spring back to the rest configuration when the force is removed, thereby retracting the piercing element from the skin.

Preferably, the fourth layer has one or more protrusions that are arranged extending over the aperture or apertures of the third layer. Ideally, the skin-piercing component is located within the protrusion. This further spaces the skin-piercing component apart from the second layer, thereby reducing the likelihood of the skin-piercing component rupturing the second layer unintentionally. The protrusion of the fourth layer may also be referred to as a "blister".

Ideally, the fourth layer, or at least the protrusion of the fourth layer, is formed from a resilient material. The protrusion of the fourth layer therefore retains its shape until it is pressed towards the second and first layers. The resiliency of the material also means the protrusion will return towards its original shape after it has been deformed, thereby functioning to draw the skin piercing component away from the second and first layers and therefore away from the skin of the recipient when in use.

Ideally, the protrusion of the fourth layer is substantially dome shaped. Preferably, the dome is flattened to provide a substantially flat upper surface.

Preferably, the protrusion of the fourth layer has an inner and outer portion. The outer portion may be vertical or almost vertical with respect to the surrounding part of the fourth layer and/or with respect to the third layer, and the inner portion may be parallel with or almost parallel with the surrounding part of the fourth layer and/or the third layer. Where the inner portion meets the outer portion is ideally gradually curved.

In one embodiment, the protrusion of the fourth layer is reinforced. This further assists in preventing accidental collapse of the protrusion and in urging the protrusion to return to its original shape after it has been deformed.

Ideally, the protrusion contains a greater amount of reinforcement towards the periphery of the protrusion than towards the centre. When the protrusion of the fourth layer is pressed towards the second and first layers, the centre will therefore deform first, with the reinforcements preventing the more peripheral part from collapsing until a threshold force is met. The collapse pattern of the protrusion is therefore predetermined. This enhances the accuracy of the direction of movement of the skin-piercing component towards the protrusion of the second layer as the protrusion of the fourth layer will almost always be collapsed at the centre first. Furthermore, by arranging the peripheral part to be more reinforced than the centre, the protrusion is thereby reinforced to prevent accidental collapse and to allow it to easily return to its default shape, without making it substantially more difficult to operate. If, in contrast, the entirety of the protrusion was reinforced by the same amount, then it could be difficult to press the protrusion towards the second and first layers in use. Selectively reinforcing certain areas of the protrusion provides the benefits of reinforcement without compromising user friendliness.

Ideally, the protrusion is reinforced by having one or more areas of increased thickness. The increased thickness may be as a result of forming the fourth layer such that it is thicker in some areas than others, or by applying separate additional material to the fourth layer to increase the overall thickness and to reinforce the fourth layer in certain areas. The increased thickness may alternatively be as a result of the protrusion having an indentation so that a part of the protrusion extends out of the surrounding plane of the protrusion, rather than there being an actual change in the material thickness of the protrusion. The protrusion in this embodiment is thereby reinforced by having an indentation.

Preferably, the protrusion of the fourth layer is reinforced by one or more circular reinforcements.

Ideally, the protrusion of the fourth layer is reinforced by a plurality of circular reinforcements.

Ideally, the circular reinforcements are coaxial.

Preferably, the circular reinforcements are of differing circumferences.

Ideally, the spacing between the circular reinforcements increases in a direction from the centre of the protrusion towards the periphery of the protrusion.

Ideally, the inner portion of the protrusion of the fourth layer corresponds generally in shape to the base of the skin-piercing component.

In an embodiment, the protrusion of the second layer is configured to receive and guide the skin-piercing component, and more specifically the piercing element of the skin-piercing component. Ideally, the protrusion has a textured surface. When the piercing element contacts the textured surface, it is less likely to be deflected off the surface than if the surface was smooth. The surface of the protrusion may be configured in other ways to guide the piercing element as it is moved towards the first layer. For example, the surface could have a substantially central indentation to urge the piercing element towards the centre of the protrusion.

In one embodiment, the first layer has an adhesive surface for contacting the skin. This ensures the skin penetration device remains in place on the recipient's skin during operation of the device. Furthermore, the adhesive surface can prevent the substance from travelling away from the site where the skin was pierced and getting between the skin and the first layer. The skin penetration device may further comprise a removable cover layer, placed such that the first layer is between the second layer and the removable cover layer. The removable cover layer thereby protects the adhesive surface of the first layer before the skin penetration device has been used.

In another embodiment, the skin penetration device comprises a fifth layer that is applied to the skin during use. In this embodiment, the first layer is located between the second and fifth layers. Ideally, the fifth layer can be adhesively attached to the skin. When the skin penetration device is operated, at least part of the skin-piercing component, most preferably the piercing element, passes through the plane of the first and fifth layers and into the recipient's skin. Ideally, the first layer is separable from the fifth layer. Therefore, after the skin penetration device has been operated, all layers other than the fifth layer can be removed, with the fifth layer remaining on the skin. Ideally, an adhesive surface or substance is present between the first layer and the fifth layer, the adhesive surface or substance being either on the first or fifth layer, or both layers.

Preferably, the skin penetration device further comprises a removable cover layer on the fifth layer, which protects the adhesive surface of the fifth layer before it is applied to the skin.

Ideally, the fifth layer has a thickness of at least 0.05 mm, and most preferably of between 0.05 and 1 mm.

Ideally, the fifth layer comprises one or more apertures. Most preferably, the fifth layer comprises one or more apertures corresponding to the location of apertures in the third layer, or corresponding to the location of the skin-piercing component, so that when the skin penetration device is operated, at least part of the skin-piercing component, most preferably the piercing element, passes through the aperture of the fifth layer.

The skin penetration device, including the fifth and first layers, may be applied to the skin of the recipient together in one step, or alternatively, the fifth layer can initially be placed on the skin, and then the remainder of the skin penetration device can be replaced on the fifth layer. Advantageously, this allows the operator to initially assess the suitability of the application site before then applying the remainder of the skin penetration device and operating said device. For example, if the operator applied the fifth layer and then realised that the skin was damaged in an area that would be pierced by the skin penetration device if operated in that area, or if the area in general was not sufficiently flat, the operator could reposition the fifth layer to a more suitable location on the recipient's skin before operating the skin penetration device.

Preferably, the fifth layer has an orientation indicator. Ideally, the orientation indicator is part of the fifth layer that extends beyond the periphery of one or more of the other layers of the skin penetration device when assembled. Ideally, the orientation indicator is present at opposing ends of the fifth layer. This enables the operator to easily align the remainder of the skin penetration device with the fifth layer when assembling the skin penetration device on the recipient and before operation of the device. Ideally, the orientation indicator is colour-coded. Ideally, one or more of the other layers, for example the second layer, has one or more indicators that can be aligned with the orientation indicator or indicators of the fifth layer. Advantageously, the operator can apply the fifth layer, then align the remainder of the skin penetration device to the fifth layer using the orientation indicators. The colour-coding ensures that the layers are correctly oriented with respect to one another. The orientation indicators may also be used to determine which site of penetration contains which test substance or concentration of substance, as it is possible to cross-reference the location of the orientation indicators on the fifth layer to the sites of penetration. This avoids the requirement of using markers to write on the recipient's skin which could be irritating to the recipient. Yet further advantageously, the thickness of the fifth layer also helps contain any excess run-off of the test substance, and the fifth layer provides a guide for photographic recording of the results.

After the remainder of the skin penetration device has been removed from the fifth layer, leaving the fifth layer on the skin of the recipient, the fifth layer can be covered by absorbent material to avoid run-off of the test substance. Further advantageously, the absorbent material prevents the risk of ingestion of the test substance by the recipient. The absorbent material may be a part of the skin penetration device and could be packaged with the skin penetration device, wherein the absorbent material is thereby a sixth layer of the skin penetration device. The absorbent material may be adhesive so that it adheres to the fifth layer and/or the skin of the recipient and it may further be hygroscopic. The absorbent material is ideally a panel of material. The absorbent material ideally has a thickness of between 2 to 6 mm.

In some embodiments, the first layer may have an increased thickness. This can further prevent the likelihood of the substance travelling away from the site where the skin was pierced.

The skin penetration device may have markings that are transferred to the skin of the recipient when it is applied to the skin. The markings can denote information so that the operator can identify which pierced part of the skin corresponds to which substance or concentration of substance that has been applied. The transferrable markings may be present on the first or fifth layers.

In an embodiment, the protrusions of the second layer and fourth layer (if present) are arranged in staggered rows. This minimalises the required amount of materials to produce the skin penetration device and improves the ease of use, because it reduces the width required for two or more rows.

In an embodiment, the skin penetration device comprises orientation indicia so that the operator can correctly orientate the device so that it is clear to the operator which substances have been applied at each site of skin piercing.

Ideally, one or more of the layers of the skin penetration device have an alignment means to align the layers with respect to one another during manufacture.

Preferably, the alignment means comprises one or more alignment holes. Ideally, the alignment hole or holes in each layer of the skin penetration device are arranged in a position that corresponds to the same position of the alignment hole or holes in the other layers of the skin penetration device. The alignment hole or holes can receive an upstanding rod. Each layer can be placed in turn with the upstanding rod extending through the alignment hole thereby aligning the layers with respect to one another.

Ideally, one or more layers comprises two or more alignment holes. Preferably, there is at least one alignment hole at or about a corner of a layer, and another alignment hole at or about a diagonally opposing corner. This is sufficient to satisfactorily align the layers while only requiring two alignment holes per layer.

According to a third aspect of the invention there is provided a skin penetration device comprising a skin-piercing component disposed within a protrusion of a layer of the skin penetration device, wherein the protrusion is reinforced. The skin penetration device may have any of the features of the skin-penetration device disclosed in relation to the first aspect of the invention.

Ideally, the protrusion is substantially dome shaped. Preferably, the dome is flattened to provide a substantially flat upper surface.

Preferably, the protrusion has an inner and outer portion.

Ideally, the protrusion contains a greater amount of reinforcement towards the periphery of the protrusion than towards the centre.

Ideally, the protrusion is reinforced by having one or more areas of increased thickness.

Preferably, the protrusion is reinforced by one or more circular reinforcements.

Ideally, the protrusion is reinforced by a plurality of circular reinforcements.

Ideally, the circular reinforcements are coaxial.

Preferably, the circular reinforcements are of differing circumferences.

Ideally, the spacing between the circular reinforcements increases in a direction from the outer portion towards the inner portion.

Ideally, the first layer has an adhesive surface.

According to a fourth aspect of the invention there is provided a skin penetration device comprising a layer having a protrusion that is configured to receive and guide a skin-piercing component. The skin penetration device may have any of the features of the skin-penetration device disclosed in relation to the first aspect of the invention.

Ideally, the protrusion has a textured surface, or a substantially central indentation to urge the piercing element towards the centre of the protrusion.

According to a fifth aspect of the invention there is provided a skin penetration device for administering a test substance to the skin of a recipient, the skin penetration device comprising:
- a first layer,
- a second layer disposed on the first layer, the second layer comprising at least one protrusion thereby providing a hollow space within the protrusion for containing a substance to be applied to the skin of the recipient, and wherein the first layer comprises an aperture and is arranged such that the protrusion of the second layer is locatable or is located within the aperture,
- a third layer disposed on the second layer, and
- a skin-piercing component, the skin-piercing component being disposed within and/or above the aperture of the first layer and between the second and third layers.

The skin penetration device may have any of the features of the skin-penetration device disclosed in relation to the first aspect of the invention. It should be noted that the first layer of the skin penetration device of the fifth aspect of the invention corresponds to the third layer in the first aspect of the invention, and the third layer in the fifth aspect corresponds to the fourth layer in the first aspect.

Ideally, the skin-piercing component has a width dimension, and the aperture of the first layer has a width dimension, wherein the width of the aperture is less than the width of the skin-piercing component. Advantageously, the skin-piercing component cannot entirely pass through the aperture of the first layer, and the thickness of the first layer serves to space the skin-piercing component away from the surface of the skin when in use.

Ideally, the second layer comprises a plurality of protrusions, the first layer comprises a plurality of corresponding apertures, and there is a corresponding plurality of skin-piercing components.

Ideally, the first layer has a thickness of between 1 and 10 mm or more preferably between 1.5 and 5 mm. Preferably, the first layer is formed of a rigid and/or non-compressible and/or non-flexible plastic. In another embodiment, the first layer is formed from a flexible and/or compressible material, most preferably, a flexible and/or compressible plastic material.

Preferably, the third layer has one or more protrusions that are arranged extending over the aperture or apertures of the first layer. Ideally, the skin-piercing component is located within the protrusion.

Ideally, the skin-piercing component is adhered to, held by, or integrally formed with the third layer. Preferably, the third layer is stretched over the skin-piercing component. Ideally, the third layer conforms to the shape of the skin-piercing component when the skin penetration device is assembled. Ideally, the third layer is formed from a resilient material. Ideally, the third layer is a film, most preferably, a breathable film. Preferably, the third layer is transparent. Ideally, the third layer is formed from Tegaderm^{®}.

According to a sixth aspect there is provided an apparatus for assisting in the assembly of a skin penetration device, the apparatus comprising one or more upstanding rods that can receive an alignment hole of a layer of a skin penetration device, such that multiple layers of the skin penetration device can be stacked with the upstanding rod extending through the alignment hole of each layer, thereby aligning the multiple layers of the skin penetration device.

According to a seventh aspect there is provided a method (not claimed) of manufacturing a skin penetration device according to the first aspect of the invention, the skin penetration device having four layers and the method comprising the following steps in any order:
- providing the fourth layer,
- setting a skin-piercing component on the fourth layer,
- setting the third layer on the fourth layer,
- setting the second layer on the third layer, and
- setting the first layer on the second layer.

Ideally, the method also comprises adhering the skin-piercing component to the fourth layer. Ideally, the method further comprises providing a substance between the first and second layers, most preferably in a protrusion of the second layer. Ideally, this step is done prior to applying the first layer such that the substance is captured between the first and second layers and most preferably at the protrusion of the second layer. The substance may be an allergenic substance and it may be in liquid form.

Ideally, the method comprises manufacturing the second layer such that the protrusion has a textured surface or other suitable configuration that guides the skin-piercing component when it is moved towards the protrusion in use.

Preferably, the method comprises manufacturing the fourth layer with at least one protrusion that is aligned with the aperture of the third layer when assembled.

Ideally, the method comprises reinforcing the protrusion of the fourth layer by producing areas of increased thickness. Preferably, the areas of increased thickness are produced by forming an indentation in the fourth layer so that a part of the protrusion extends beyond the plane of the protrusion.

Ideally, the method comprises providing a removable cover layer, covering the first layer such that the first layer is between the removable cover layer and second layer.

The method may involve applying markings to the skin penetration device.

Preferably, the method comprises applying orientation indicators to the skin-piercing device, or forming the skin-piercing device such that it has orientation indicators, so that it can be orientated correctly in use.

Ideally, the method comprises forming alignment means in the layers, most preferably, alignment holes.

Preferably, the method comprises arranging each layer in turn on an apparatus that extends through the alignment means, or alignment hole or holes of the layers, such that the apparatus serves to correctly align the layers with respect to one another.

Ideally, after the second to fourth layers have been assembled, the method comprises removing any apparatus that is extending through the alignment hole or holes, and then applying the first layer to the second layer. The substances may be added to the protrusion or protrusions of the second layer before or after the apparatuses extending through the alignment hole or holes have been removed.

Ideally, the method comprises manufacturing the skin-piercing component by providing a base and punching out a part of the base to produce the piercing element.

According to an eighth aspect there is provided a method of manufacturing (not claimed) a skin penetration device according to the fifth aspect of the invention, the skin penetration device having three layers and the method comprising the following steps in any order:
- providing the third layer,
- setting a skin-piercing component on the third layer or arranging the third layer around a skin-piercing component,
- setting the second layer on the third layer,
- setting the first layer on the second layer.

The invention will now be described, by way of example only, with reference to the accompanying drawings in which: -
Figure 1 is a perspective view of an embodiment of a skin penetration device according to the first aspect of the invention.
Figure 2 is a top elevation view of the skin penetration device of Figure 1.
Figure 3 is a side elevation view of the skin penetration device of Figure 1.
Figure 4 is an exploded perspective view of the skin penetration device of Figure 1.
Figure 5 is an exploded side view of the skin penetration device of Figure 1.
Figure 6 is a side cross-section view of the skin penetration device of Figure 1, the cross-section being along the axis A-A as shown in Figure 2.
Figure 7 is an enlarged view of the encircled part B of Figure 6.
Figure 8 is a perspective view of a skin-piercing component according to the invention.
Figure 9 is a perspective view of a further embodiment of a skin penetration device according to the first aspect of the invention.
Figure 10 is a top elevation view of the skin penetration device of Figure 9.
Figure 11 is a side elevation view of the skin penetration device of Figure 9.
Figure 12 is a side cross-section view of the skin penetration device of Figure 9, the cross section being along the axis A-A as shown in Figure 10.
Figure 13 is an enlarged view of the encircled part B of Figure 12.
Figure 14 is a perspective view of a further embodiment of a skin penetration device according to the first aspect of the invention.
Figure 15 is a top elevation view of the skin penetration device of Figure 14.
Figure 16 is a side elevation view of the skin penetration device of Figure 14.
Figure 17 is an exploded perspective view of the skin penetration device of Figure 14.
Figure 18 is a side cross-section view of the skin penetration device of Figure 14, the cross section being along the axis A-A as shown in Figure 15.
Figure 19 is an enlarged view of the encircled part B of Figure 18.
Figure 20 is a further enlarged view of Figure 19.
Figure 21 is a top elevation view of a further embodiment of a skin penetration device according to the fifth aspect of the invention.
Figure 22 is a side elevation view of the skin penetration device of Figure 21.
Figure 23 is an enlarged cross-section view of part of the skin penetration device of Figure 21.
Figure 24 is an exploded side view of the skin penetration device of Figure 21.
Figure 25 is an exploded perspective view of the skin penetration device of Figure 21.

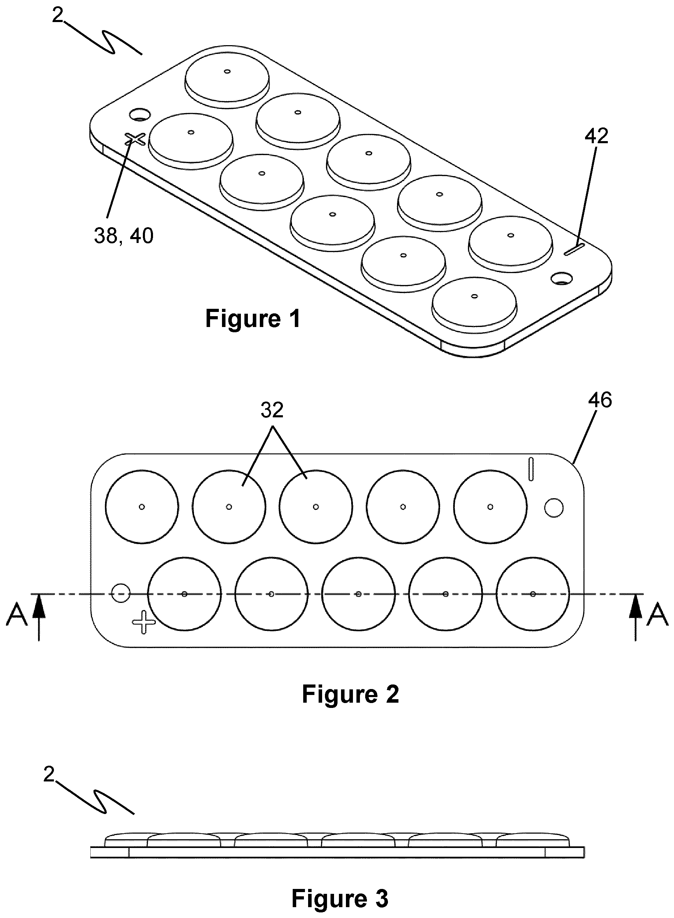

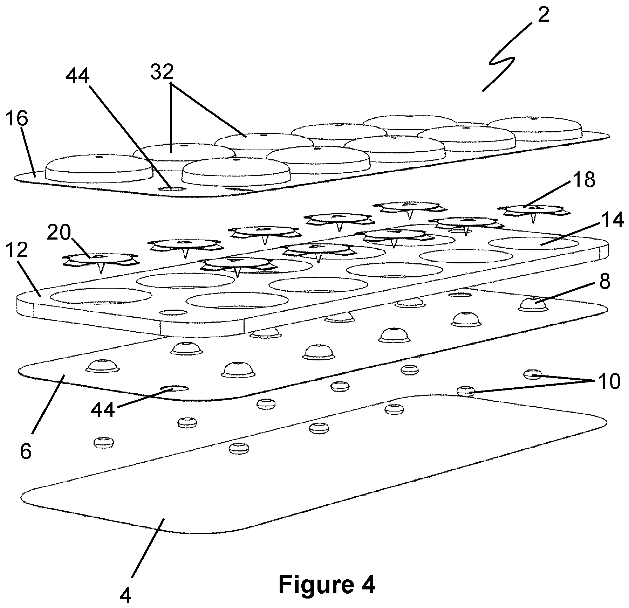

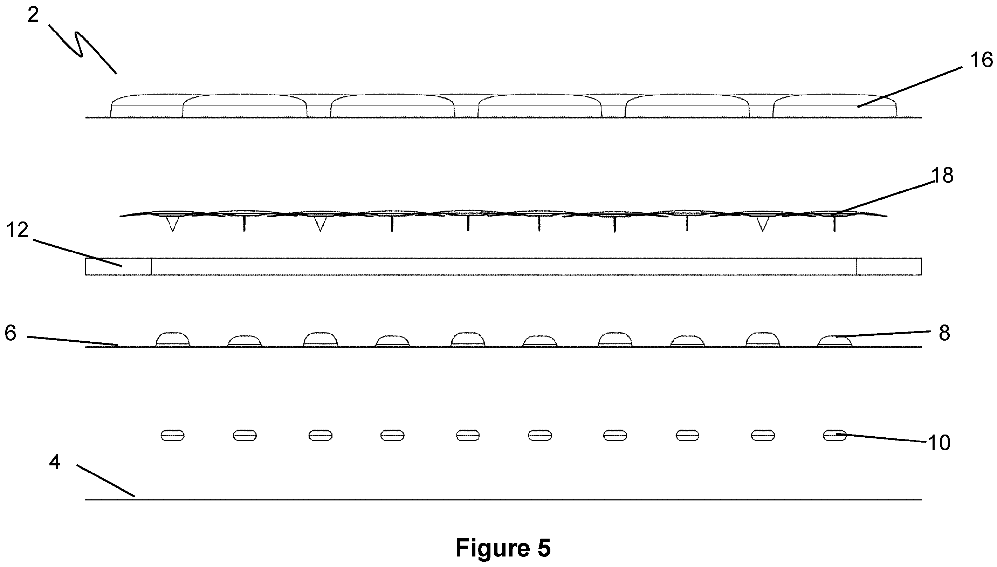

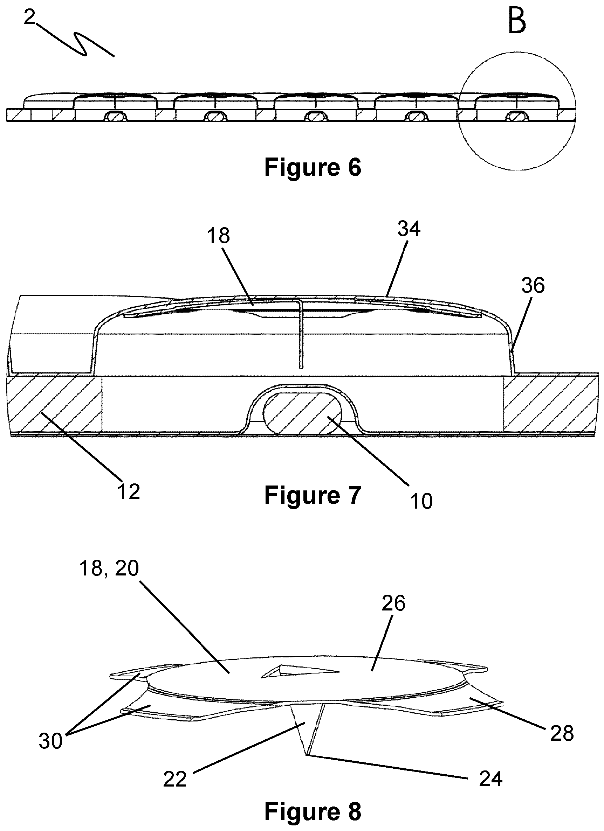

Referring to Figures 1 to 7 there is shown a skin penetration device indicated generally by reference numeral 2. The skin penetration device 2 has a first layer 4 which is placed on the skin of a recipient in use. A second layer 6 is disposed on the first layer 4. In this embodiment, both the first layer 4 and second layer 6 are formed from polyvinylidene dichloride (PVDC), although other materials may be suitable. The first and second layers 4, 6 each have a thickness of between 0.07 and 0.12 mm. Specifically, the first layer 4 has a thickness of 0.075 mm and the second layer 6 has a thickness of 0.12 mm, although variations in this thickness may also be used. The second layer 6 has ten protrusions 8 which protrude relative to the first layer 4, thereby providing a space between the first and second layers 4, 6 for containing a substance 10 to be applied to the skin of the recipient. In this embodiment, the protrusion 8 of the second layer 6 is a domed protrusion. In other embodiments not shown, the second layer may have more or fewer than ten protrusions. The skin penetration device 2 further has a third layer 12 which is disposed on the second layer 6. The third layer is formed from a rigid plastic in this embodiment. The third layer 12 has apertures 14 that correspond to the protrusions 8 of the second layer 6, with the protrusions 8 being located within the apertures 14 when assembled. The skin penetration device 2 further has a fourth layer 16 disposed on the third layer 12 and covering the apertures 14 of the third layer 12. In this embodiment, the fourth layer 16 is formed from polyvinyl chloride (PVC), although other materials may be suitable.

A skin-piercing component 18 is disposed such that is located above the protrusion 8 of the second layer 6. More specifically, the skin-piercing component 18 is located between the second and fourth layers 6, 16. In this embodiment, the skin-piercing component 18 is located above the aperture 14 of the third layer 12 when not in use. In other embodiments not shown, the skin-piercing component 18 may extend into the aperture 14 when not in use. The skin penetration device 2 in the particular illustrated embodiment has ten protrusions 8 of the second layer 6, ten corresponding apertures 14 in the third layer 12 and ten skin-piercing components 18. The skilled person will understand that the number of protrusions, apertures and skin-piercing components may be adjusted as desired. The first and second layers 4, 6 are formed from polyvinylidene chloride (PVDC) but other suitable materials may be used as desired. The skin penetration device 2 has rounded corners 46 when assembled.

The skin-piercing component 18 is adhered to the fourth layer 16 and this retains the skin-piercing component 18 in a position that is spaced apart from the protrusion 8 of the second layer 6, thereby preventing the skin-piercing component 18 from unintentionally penetrating the second layer 6, for example, during transportation of the skin penetration device 2. Further, the thickness of the third layer 12 functions to space the fourth layer 16, and therefore the skin-piercing component 18, apart from the protrusion 8 of the second layer 6. This again prevents the skin-piercing component 18 from unintentionally penetrating the second layer 6. The thickness of the third layer 12 also provides the skin penetration device 2 with improved structural integrity and rigidity, thereby making it even less likely that the device may be damaged, for example, during transport. The third layer has a thickness of 3 mm but in other embodiments it may have a smaller or greater thickness than 3 mm. A range of thicknesses between 1.5 and 5 mm is suitable.

The skin-piercing component 18 has a base 20 and a piercing element 22 extending from the base 20. In other embodiments, the skin-piercing component could have a plurality of piercing elements extending from the base. The piercing element 22 has a pointed end 24 and is connected to the base 20 of the skin-piercing component 18 at the other end opposing the pointed end 24. When assembled, the base 20 is oriented such that it is abutting the fourth layer 16, with the piercing element 22 extending towards the protrusion 8 of the second layer 6. The skin-piercing component 18 is deformable. In particular, it has a rest configuration and is configured to be biased towards the rest configuration such that it may be deformed but returns to the rest configuration when the deforming force is removed. Accordingly, if the skin-piercing component 18 is deformed when it is pressed into the recipient's skin, on removal of the deforming force, the skin-piercing component 18 will revert to the rest configuration. When doing so, this can draw the piercing element 22 out of the skin, thereby preventing the piercing element 22 from remaining in the recipient's skin for any longer than necessary and preventing the need to manually urge the piercing element 22 out of the skin.

In more detail, the skin-piercing component 18 is configured to be deformed when it is pressed against a substantially flat surface, such as the skin of a recipient. The piercing element 22 is located substantially centrally on the base 20 of the skin-piercing component 18, and the base has an inner portion 26 and outer portion 28; the piercing element 22 being connected to the base 20 at the inner portion 26. The outer portion 28 is deflected towards the pointed end 24 of the piercing element 22, and towards the second layer 6 when the skin penetration device 2 is assembled. The base 20 slopes downwardly away from where it is connected to the piercing element 22, the slope extending in a direction towards the pointed end 24 of the piercing element 22, or, to put another way, towards the axis of the piercing element 22 or towards the second layer 6 when assembled.

The base 20 is substantially dome shaped, with the piercing element 22 arranged extending from the top of the dome past the bottom of the dome. The domed shape of the base 20 is less than a hemisphere and is similar to a "saucer dome" in shape. The base 20 of the skin-piercing component 18 is configured to enable the base 20 to move between the dome shape in the rest configuration to a flattened configuration. In this embodiment, the outer portion 28 has four flanges 30 that extend out from the inner portion 26. The spacing between the flanges 30 enables the base 20 to be moved between the flattened and rest configurations. In other embodiments not shown, the outer portion may have any suitable desired number of flanges, or the base of the skin-piercing component may have one or more incisions or cut-outs to enable it to be flattened. The skilled person will understand that the incisions or cut-outs need not be formed by physically cutting the base but may instead be formed as part of the design of the base during manufacture.

The piercing element 22 is formed by punching out a part of the base 20 of the skin-piercing component 18 and bending the punched-out part away from the base 20. The piercing element 22 is generally triangular in shape. The skin-piercing component 18 is formed from spring steel, most preferably stainless steel. The skin-piercing component 18 is configured to provide tactile feedback to the user when sufficient force has been applied to the component 18. In more detail, the skin-piercing component 18 is arranged to spring from the rest configuration to a flattened configuration when a sufficient flattening force has been applied. This sudden change in configuration when the skin-piercing component 18 is pressed towards the skin provides the person administering the device with tactile feedback and assures the person that sufficient force has been applied and the skin-piercing component 18 has pierced the skin. As the skin-piercing component 18 is biased towards the rest configuration, it will spring back to the rest configuration when the force is removed, thereby retracting the piercing element 22 from the skin.

The fourth layer 16 has one or more protrusions 32 that protrude relative to the third layer 12 and are arranged extending over the apertures of the third layer 12. This further spaces the skin-piercing component 18 apart from the second layer 6, thereby reducing the likelihood of the skin-piercing component 18 rupturing the second layer 6 unintentionally. The fourth layer 16 is formed from a resilient material and therefore the protrusions 32 retain their shape until the protrusion 32 is pressed towards the second and first layers 4, 6. The resiliency of the material also means the protrusion 32 will return towards its original shape after it has been deformed, thereby functioning to draw the skin piercing component 18 away from the second and first layers 4, 6 and therefore away from the skin of the recipient when in use. The protrusion 32 of the fourth layer 16 is dome shaped, having a substantially flat upper surface. The protrusion 32 of the fourth layer 16 has inner and outer portions 34, 36. The outer portion 36 is almost vertical with respect to the surrounding part of the fourth layer 16 and with respect to the third layer 12, and the inner portion 34 is almost parallel with the surrounding part of the fourth layer 16 and the third layer 12. Where the inner portion 34 meets the outer portion 36 is gradually curved. The inner portion 34 of the protrusion 32 of the fourth layer 16 corresponds generally in shape to the base 20 of the skin-piercing component 18.

The first layer 4 has an adhesive surface for contacting the skin. This ensures the skin penetration device 2 remains in place on the recipient's skin during operation of the device. Furthermore, the adhesive surface can prevent the substance from travelling away from the site where the skin was pierced and getting between the skin and the first layer. The skin penetration device 2 may further comprise a removable cover layer (not shown), placed such that the first layer 4 is between the second layer 6 and the cover layer, to protect the adhesive surface of the first layer 4 before the skin penetration device 2 has been used.

In other embodiments not shown, the first layer may have an increased thickness. This can further prevent the likelihood of the substance travelling away from the site where the skin was pierced. The first layer 4 has markings (not shown) that are transferred to the skin of the recipient when the first layer 4 is applied to the skin. The markings can denote information so that the operator can identify which pierced part of the skin corresponds to which substance or concentration of substance that has been applied.

The protrusions 8, 32 of the second and fourth layers 4, 16 are arranged in staggered rows. In this particular embodiment, the protrusions 8 of the second layer 6 have differing sizes. In particular, one row of protrusions 8 has a diameter of 7 mm whereas the other row has a diameter of 5 mm. This facilitates use of different amounts of substances to be applied to the recipient's skin.

The skin penetration device 2 further has orientation indicia 38 so that the operator can correctly orientate the device 2 so that it is clear to the operator which substances have been applied at each site of skin piercing. In particular, the orientation indicia are a plus sign 40 and a minus sign 42 arranged on the fourth layer 16. The plus sign 40 is arranged proximal one corner of the skin penetration device 2, and the minus sign 42 is arranged at a diagonally opposing corner.

The second, third and fourth layers 6, 12, 16 of the skin penetration device 2 have an alignment means to align the layers with respect to one another during manufacture. In further detail, the alignment means comprises ideally at least two alignment holes 44 per layer, as shown in the illustrated embodiment. Some degree of alignment may be obtained with a single alignment hole, but having at least two spaced apart alignment holes substantially improves the accuracy of the alignment. The alignment holes 44 in each layer of the skin penetration device 2 are arranged in a position that corresponds to the same position of the other layers of the skin penetration device 2. The alignment holes 44 can receive an upstanding rod. Each layer can be placed in turn with the upstanding rod extending through the alignment hole 44 thereby aligning the layers with respect to one another. In the illustrated embodiment, one alignment hole 44 is located near one corner of each of the second, third and fourth layers 6, 12, 16, and another alignment hole 44 is located near a diagonally opposing corner.

In use, the operator removes a cover layer to expose the adhesive first layer 4. The first layer 4 is then set onto the skin of the recipient and pressed to adhere the skin penetration device 2 to the skin. The operator then sequentially presses each of the protrusions 32 of the fourth layer 16 towards the skin of the recipient. In doing so, the skin-penetrating component 18 is moved towards the protrusion 8 of the second layer 6, and the protrusion 8 is ruptured by the piercing element 22. The piercing element 22 then travels through the substance 10 and then pierces through the first layer 4 and into the skin of the recipient. The operator continues pressing the skin-piercing component 18 towards the skin until haptic feedback, resulting from the skin-piercing component 18 springing from the rest configuration to the flattened configuration, is received. This occurs because the outer portion 28 of the skin-piercing component 18 abuts the second layer 6 and the base 20 is thereby flattened. This configuration also limits the depth of penetration of the piercing element 22 because the base 20 prevents the piercing element 22 from being inserted any further when the base 20 abuts the second layer 6. When the force pressing the skin-piercing component 18 into the skin is removed, the base 20 of the skin-piercing component 18 reverts to the dome-shaped rest configuration thereby drawing the piercing element 22 out of the skin.

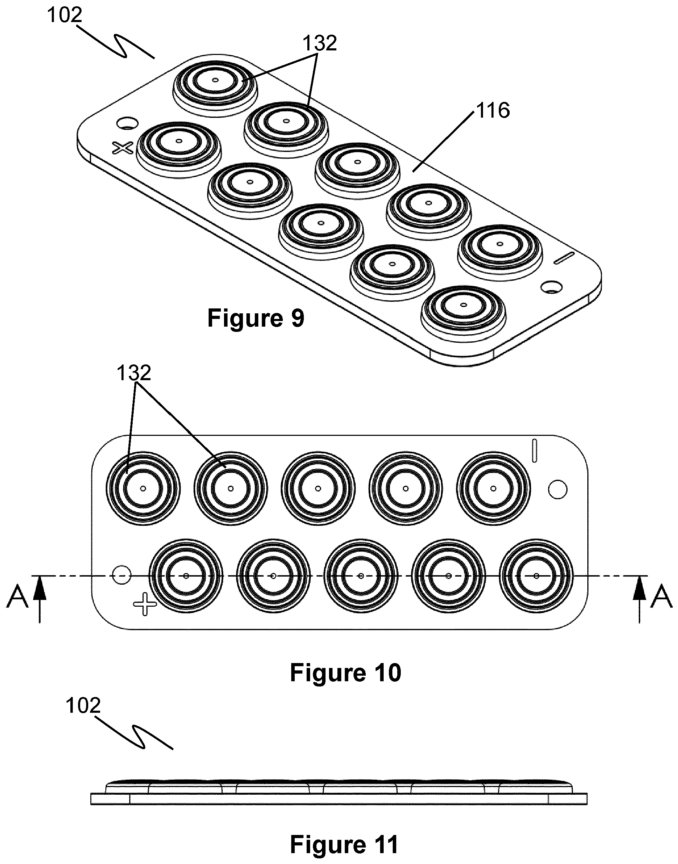

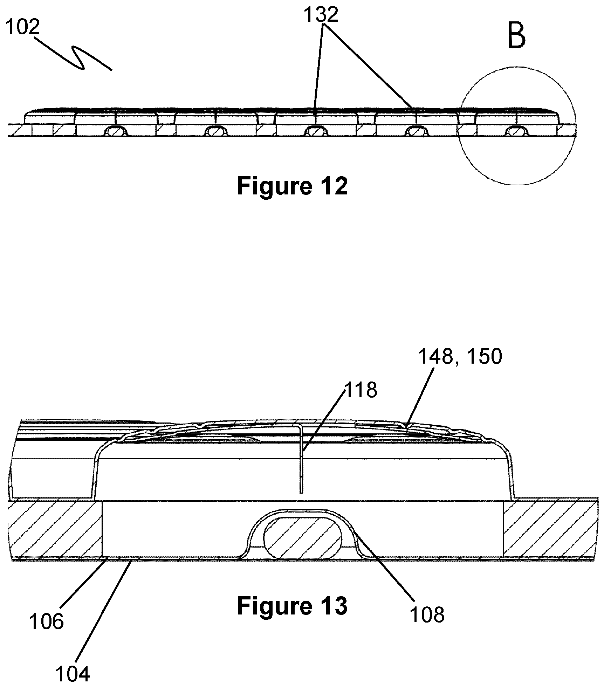

In Figures 9 to 13 there is shown a further embodiment of a skin penetration device indicated generally by reference numeral 102. In this embodiment, the protrusions 132 of the fourth layer 116 are reinforced. There is a greater amount of reinforcement towards the periphery of the protrusion 132 than towards the centre. When the protrusion 132 of the fourth layer 116 is pressed towards the second and first layers 104, 106, the centre will therefore deform first, with the reinforcements preventing the more peripheral part from collapsing until a threshold force is met. The collapse pattern of the protrusion 132 is therefore predetermined. This enhances the accuracy of the direction of movement of the skin-piercing component 118 towards the protrusion 108 of the second layer 106 as the protrusion 132 will almost always be collapsed at the centre first. Furthermore, by arranging the peripheral part to be more reinforced than the centre, the protrusion 132 is thereby reinforced to prevent accidental collapse and to allow it to easily return to its default shape, without making it substantially more difficult to operate. If, in contrast, the entirety of the protrusion 132 was reinforced by the same amount, then it could be difficult to press the protrusion towards the second and first layers 104, 106 in use. Selectively reinforcing certain areas of the protrusion 132 provides the benefits of reinforcement without compromising user friendliness.

In the illustrated embodiment, the protrusion 132 is reinforced by having indentations 148. The indentation 148 extends out of the surrounding plane of the protrusion 132. In other embodiments not shown, the protrusion may be reinforced by increasing the material thickness of the protrusion, either by forming the fourth layer such that it is thicker in some areas than others, or by applying separate additional material to the fourth layer to increase the overall thickness and to reinforce the fourth layer in certain areas. The protrusion 132 is reinforced by three circular, coaxial reinforcements 150. The axis of the reinforcements 150 is also coaxial with the protrusion 132. The spacing between the circular reinforcements 150 increases in a direction from the centre of the protrusion 132 towards the periphery of the protrusion 132.

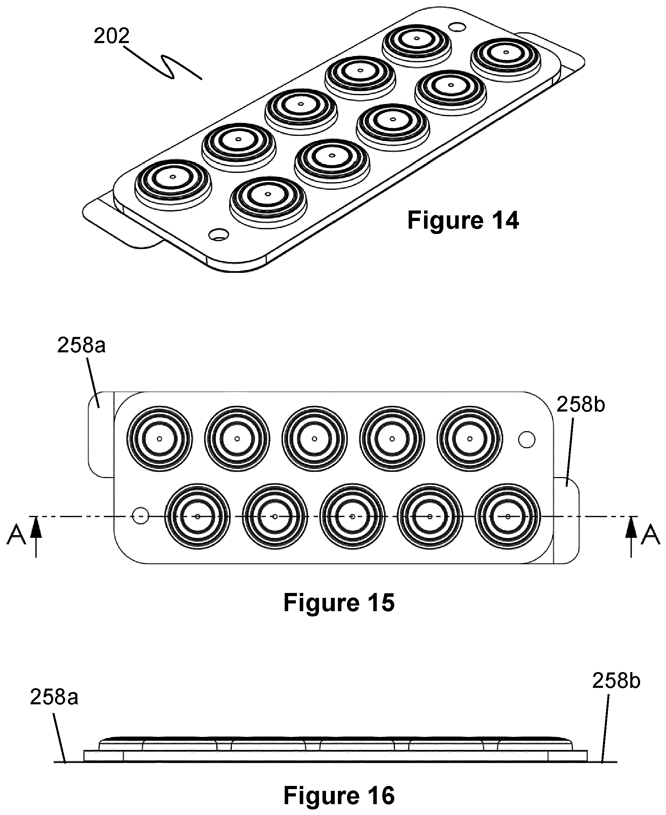

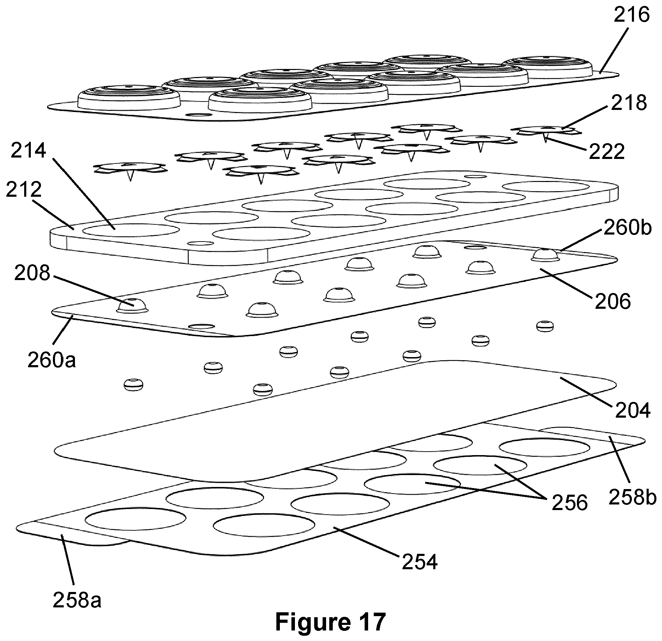

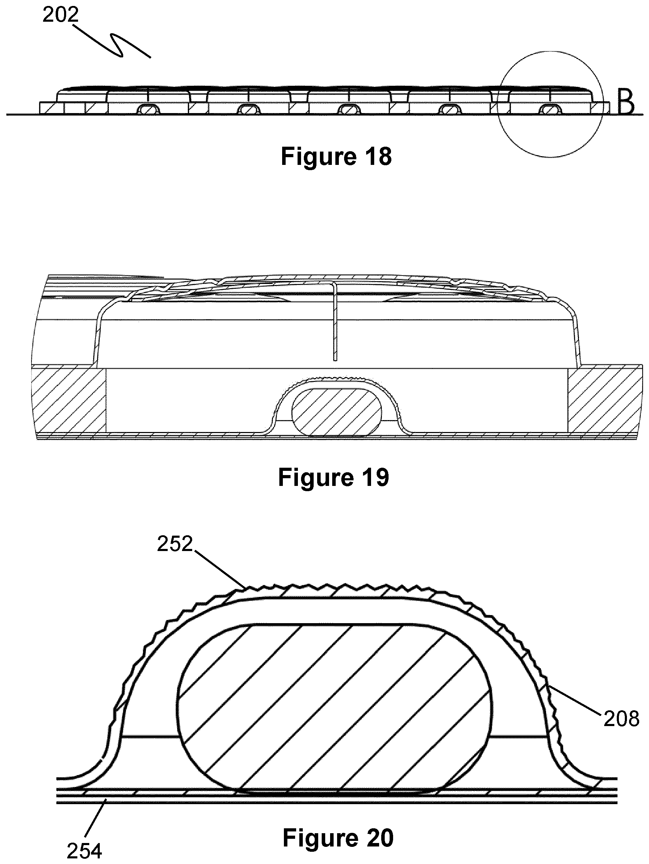

In Figures 14 to 20 there is shown a further embodiment of a skin penetration device indicated generally by reference numeral 202. In this embodiment, the protrusion 208 of the second layer 206 is configured to receive and guide the skin-piercing component 218, and more specifically the piercing element 222 of the skin-piercing component 218. The protrusion 208 has a textured surface 252 (see Figure 20). When the piercing element 222 contacts the textured surface 252, it is less likely to be deflected off the surface than if the surface was smooth. The surface of the protrusion 208 may be configured in other ways to guide the piercing element as it is moved towards the first layer 204. For example, the surface could have a substantially central indentation to urge the piercing element 222 towards the centre of the protrusion 208.

In this embodiment, the skin penetration device 202 further comprises a fifth layer 254 that is applied to the skin during use and can be adhesively attached to the skin. When the skin penetration device 202 is operated, the piercing element 222 passes through the plane of the first and fifth layers 204, 254 and into the recipient's skin. The first layer 204 is separable from the fifth layer 254. Therefore, after the skin penetration device 202 has been operated, all layers other than the fifth layer 254 can be removed, with the fifth layer 254 remaining on the skin. The first layer 204 and fifth layer 254 are connected to one another, in this embodiment, by the first layer 204 having an adhesive surface that faces the fifth layer 254. Alternatively, the adhesive surface may be present on the fifth layer 254 on the surface that faces the first layer 204, or both the first and fifth layers 204, 254 may have adhesive surfaces that contact one another when the skin penetration device 202 is assembled. Yet further alternatively, an adhesive substance could be applied on either or both the first and fifth layers 204, 254 that then adhesively and releasably attaches the first and fifth layers 204, 254 when they are pressed together. In this embodiment, the fifth layer 254 has a thickness of 0.1 mm but the skilled person will appreciate any suitable thickness of fifth layer 254 may be used.

The fifth layer 254 has ten apertures 256. The apertures 256 correspond to the location of the protrusions 208 in the second layer 206, the apertures 214 in the third layer 212, and to the location of the skin-piercing component 218, so that when the skin penetration device 202 is operated, the piercing element 222 passes through the aperture 256 of the fifth layer 254. It will be understood that the fifth layer 254 may have more or fewer apertures 256 as desired.

The skin penetration device 202, including the fifth and first layers 254, 204, may be applied to the skin of the recipient together in one step, or alternatively, the fifth layer 254 can initially be placed on the skin, and then the remainder of the skin penetration device 202 can be replaced on the fifth layer 254. This allows the operator to initially assess the suitability of the application site before then applying the remainder of the skin penetration device 202 and operating said device.

The fifth layer 254 has an orientation indicator 258a, 258b. In this embodiment, the orientation indicator 258a, 258b is part of the fifth layer 254 that extends beyond the periphery of the other layers of the skin penetration device 202 when assembled. In this embodiment, the orientation indicator 258a, 258b comprises two tabs 258a, 258b. The first tab 258a is located at one longitudinal end corner of the fifth layer 254 and the second tab 258b is located at the diagonally opposing longitudinal end corner of the fifth layer 254. This enables the operator to easily align the remainder of the skin penetration device 202 with the fifth layer 254 when assembling the skin penetration device 202 on the recipient and before operation of the device 202. Whereas a single tab would be possible, two tabs are preferable. The orientation indicator 258a, 258b is further colour-coded in this embodiment. In particular in this embodiment, the first tab 258a is blue and the second tab 258b is red. Any desirable colour scheme may be used, or differing textures or shapes could be used to indicate the orientation.

In this embodiment, the second layer 206 has corresponding indicators 260a, 260b that can be aligned with the orientation indicators 258a, 258b of the fifth layer. The indicators 260a, 260b on the second layer 206 are visible by looking at the underside of the skin penetration device 202. The second layer 206 indicators involve a first indicator 260a that is located at one longitudinal end and is colour coded to match the colour of the orientation indicator 258a on the fifth layer 254. The second layer 206 further has a second indicator 260b that is located at the opposing longitudinal end to the first indicator 260a and is colour coded to match the second orientation indicator 258b of the fifth layer 254. The operator can thereby check that the skin penetration device 202 is correctly orientated relative to the fifth layer 254 before applying to the skin. In other embodiments, the indicators may be located on other layers or on multiple layers, such as the first, third or fourth layers, 204, 212, 216. The orientation indicators 258a, 258b may also be used to determine which site of penetration contains which test substance or concentration of substance, as it is possible to cross-reference the location of the orientation indicators to the sites of penetration after use of the skin penetration device 202.

The skin penetration device 202 further has a sixth layer (not shown). The sixth layer is formed from absorbent material and is placed on the surface of the fifth layer 254 when the remaining part of the skin penetration device 202 has been removed (i.e., the first, second, third and fourth layers 204, 206, 212, 216). The sixth layer avoids run-off of the test substance and is a panel of hygroscopic material with a thickness of between 2 and 6 mm. Any suitable material and thickness of material may be used as the sixth layer as desired.

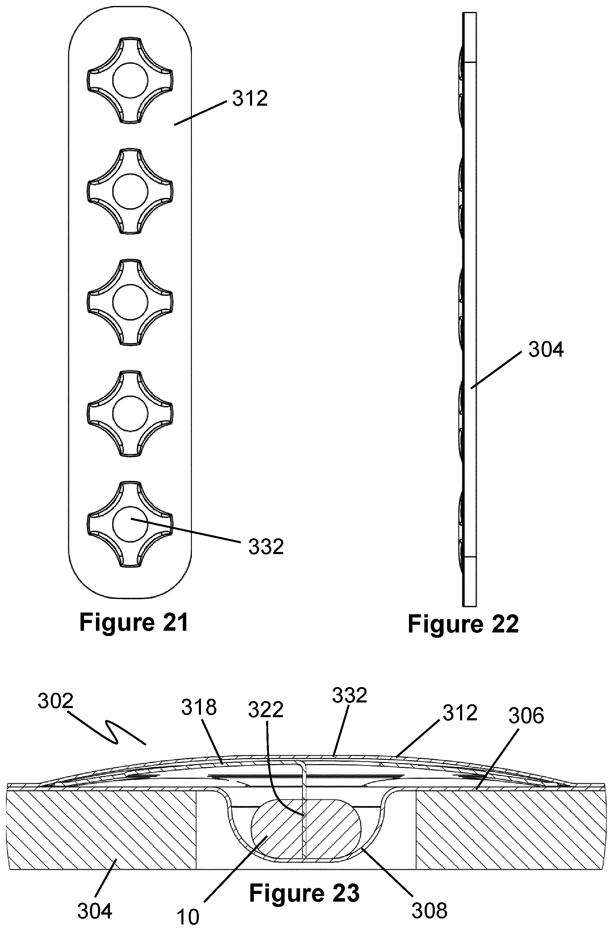

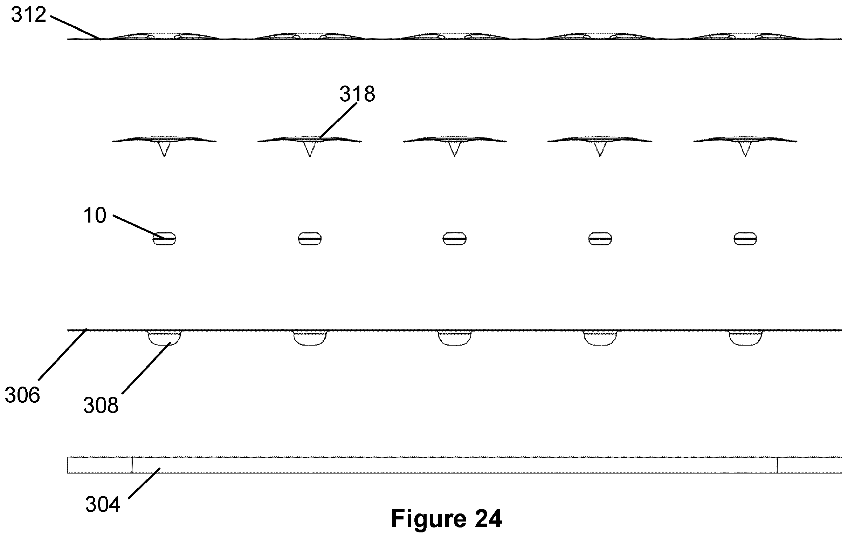

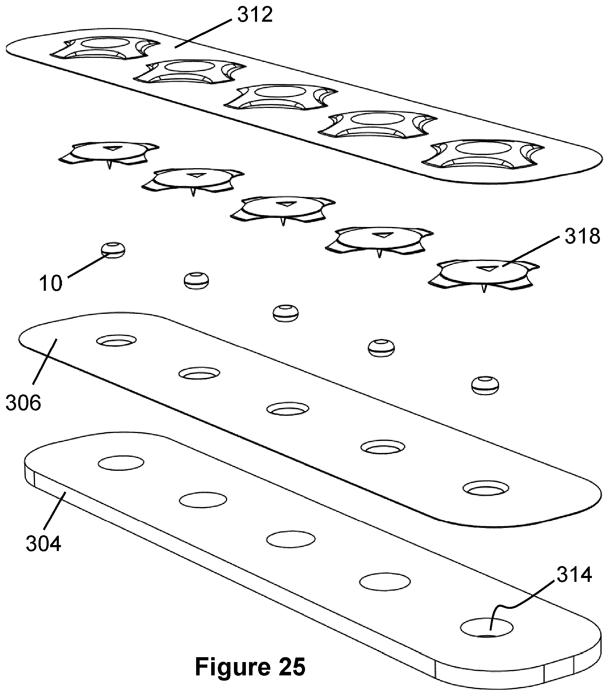

Referring now to Figures 21 to 25, there is shown a skin penetration device according to a further embodiment of the invention, indicated generally by reference numeral 302. The skin penetration device has a first layer 304 and a second layer 306. The second layer 306 is disposed on the first layer 304. The second layer 306 has five protrusions 308. In alternative embodiments, there may be more or less than five protrusions. The protrusions 308 provide a hollow space for containing a substance to be applied to the skin of the recipient.

The first layer 304 has five apertures 314, each aperture being arranged such that a protrusion 308 of the second layer 306 is located within the aperture 314. The number of apertures 314 corresponds to the number of protrusions 308. In alternative embodiments, there may be more or less than five apertures.

The skin penetration device 302 further has a third layer 312 disposed on the second layer. There is also a skin-piercing component 318 located within and above each aperture 308 and between the second and third layers 306, 312. The skin-piercing component 318 has a width dimension, and the aperture 308 of the first layer has a width dimension, wherein the width of the aperture 308 is less than the width of the skin-piercing component 318.

The first layer has a thickness of 3 mm but in other embodiments it may have a smaller or greater thickness than 3 mm. A range of thicknesses between 1.5 and 5 mm is suitable. The first layer is formed of a flexible and compressible material, but it could alternatively be formed of a non-compressible or non-flexible material. The material is resilient such that it will return to its original shape after a compressive force has been removed.

The third layer 312 has protrusions 332 that extend over the apertures 314 of the first layer 304. The protrusions 332 are formed by the shape of the skin-piercing component 318, which is located within the protrusions 332. The third layer 312 is stretched over the skin-piercing components 318 and this thereby secures the skin-piercing components 318 to the third layer 312. The third layer 312 thereby conforms to the shape of the skin-piercing component when the skin penetration device 302 is assembled. The third layer is formed from Tegaderm^{®} which is a breathable, transparent film. Any other suitable brand of film may alternatively be used, and it is optional that the film is breathable and transparent.

A substance 10 is located in the protrusion 308 of the second layer 306. The skin-piercing component 318 has a piercing element 322 which may extend into the substance 10, even before the skin penetration device 302 is in use. The piercing element 332 is thereby coated by the substance 10 which can improve efficacy of the test.

In use, the skin penetration device 302 is set on the surface of skin to be tested. The device 302 is then operated by pressing the protrusion 332 towards the skin. The piercing element 322 ruptures the second layer 306 thereby releasing the substance 10. The piercing element 322 then pierces the skin which allows the substance to enter to the skin. The skin piercing component 318 will then invert when sufficient force is applied. When the force is removed, the skin piercing component 318 will spring back to its resting configuration and at the same time will draw the piercing element 322 away from the skin, so that it is not in the skin any longer than necessary. During use, the first layer 304 will also become compressed when the skin piercing component 318 is pressed towards the skin, as the first layer 304 is located between the skin piercing component 318 and the skin. When the compressive force is removed, the first layer 304 will expand again and this can also serve to draw the piercing element 322 away from the skin. The boundary of the aperture 314 of the first layer 304 acts as a barrier to prevent the substance 10 from rolling away from the puncture site on the skin, caused by the piercing element 322 and this also serves to improve efficacy. The skin penetration device 302 can be held on the skin for as long as necessary, then removed, and the substance 10 can be wiped away from the skin.

The skin penetration device 2 can be manufactured by providing the fourth layer 16 initially in an orientation that is inverted relative to how it will be applied in use. Then the skin-piercing components 18 are adhered to the fourth layer 16 with an adhesive substance. In other embodiments, the skin-piercing components 18 could be attached to the fourth layer 16 by other means such as welding, or RF bonding. Next, the third layer 12 is set on the fourth layer 12, then the second layer 6 is set on third layer. The third layer 12 may be placed on the fourth layer 16 before the skin-piercing components 18 have been adhered to the fourth layer 16. At this stage, it is possible to introduce the substances into the protrusions 8 of the second layer 6. Then the first layer 4 is applied to the second layer. It would also be possible to manufacture the skin penetration device 2 by first manufacturing the first and second layers 4, 6 with the test substances in one step, then manufacturing the third and fourth layers 12, 16 along with the skin-piercing component 18 in another step, then combining the layers to provide the skin penetration device. With the embodiment in Figures 14 to 20, the manufacturing process includes the step of providing a fifth layer 254.

The skin penetration device 2 can be manufactured using upstanding rods (not shown) that can be inserted through the alignment holes 44 to assist in aligning the layers 6, 12, 16 during assembly. The method further comprises manufacturing the fourth layer 16 with protrusions 32 that are aligned with the aperture 14 of the third layer 12 when assembled. The method may also involve providing a removable cover layer (not shown), covering the first layer 6 such that the first layer 4 is between the cover layer and second layer 6. The method comprises manufacturing the skin-piercing component 18 by providing base 20 and punching out a part of the base 20 to produce the piercing element 22. With the embodiment in Figures 14 to 20, the method also comprises manufacturing the second layer 206 such that the protrusion 208 has a textured surface or other suitable configuration that guides the skin-piercing component 218 when it is moved towards the protrusion 208 in use.

The method further comprises applying markings (not shown) to the first layer 6, or producing the first layer 6 such that it has markings. The method comprises applying orientation indicators 38, and in particular in this embodiment, plus and minus signs 40, 42, to the skin-piercing device 2 so that it can be easily orientated correctly in use. The method also comprises forming alignment means in the layers 6, 12, 16, most preferably, alignment holes 44. These may be formed by punching out a part of the layers 6, 12, 16. With the embodiment in Figures 14 to 20, the method comprises providing orientation indicators on the fifth layer 254 as tabs 258a, 258b and indicators 260a, 260b on the second layer 206.

Regarding skin penetration device 102, the method comprises reinforcing the protrusion 132 of the fourth layer 116 by indenting parts of the protrusion 132. In particular, the method comprises forming three circular reinforcements 150 by indenting the protrusion 132. The circular reinforcements 150 are coaxial with one another and with the protrusion.

The skilled person will appreciate that all preferred or optional features of the invention described with reference to only some aspects or embodiments of the invention may be applied to all aspects of the invention.

It will be appreciated that optional features applicable to one aspect of the invention can be used in any combination, and in any number. Moreover, they can also be used with any of the other aspects of the invention in any combination and in any number. This includes, but is not limited to, the dependent claims from any claim being used as dependent claims for any other claim in the claims of this application.

In relation to the detailed description of the different embodiments of the invention, it will be understood that one or more technical features of one embodiment can be used in combination with one or more technical features of any other embodiment where the transferred use of the one or more technical features would be immediately apparent to a person of ordinary skill in the art to carry out a similar function in a similar way on the other embodiment.

The features disclosed in the foregoing description or the following drawings, expressed in their specific forms or in terms of a means for performing a disclosed function, or a method or a process of attaining the disclosed result, as appropriate, may separately, or in any combination of such features be utilised for realising the invention in diverse forms thereof.

## Claims

1. A skin-piercing component (18, 118, 218, 318) of a skin penetration device, wherein the skin-piercing component (18) has a base (20) and one or more piercing elements (22, 222, 322) extending from the base (20), and wherein the skin-piercing component (18, 118, 218, 318) has a rest configuration and is configured to be biased towards the rest configuration such that it may be deformed but returns to the rest configuration when the deforming force is removed, and **characterised in that** the one or more piercing elements (22, 222, 322) are formed by punching out a part of the base (20) of the skin-piercing component (18, 118, 218, 318) and bending the punched-out part away from the base (20).

2. A skin-piercing component (18, 118, 218, 318) as claimed in claim 1 wherein the base (20) is substantially dome shaped, and wherein the piercing element (22, 222, 322) is arranged extending from the top of the dome in a direction past the bottom of the dome, and wherein the skin-piercing component (18, 118, 218, 318) is configured to provide tactile feedback to the user when sufficient force has been applied to the component (18, 118, 218, 318).

3. A skin-piercing component (18, 118, 218, 318) as claimed in claim 2 wherein the base (20) is configured to enable the base (20) to move between the dome shape in the rest configuration to a flattened or inverted configuration, said base (20) having one or more incisions or cut-outs to enable it to be flattened or inverted, the skin-piercing component (18, 118, 218, 318) being arranged to spring from the rest configuration to a flattened or inverted configuration when a sufficient force has been applied, wherein the sudden change in configuration when the skin-piercing component (18, 118, 218, 318) is pressed towards the skin provides the person administering the device with tactile feedback and assures the person that sufficient force has been applied and the skin-piercing component (18, 118, 218, 318) has pierced the skin.

4. A skin-piercing component (18, 118, 218, 318) as claimed in any preceding claim wherein the base (20) has an inner portion (26) and an outer portion (28), and wherein the outer portion (28) is formed of a plurality of flanges (30) extending out from the inner portion (26), and wherein the piercing element (22, 222, 322) is connected to the base (20) at the inner portion (26).

5. A skin-piercing component (18, 118, 218, 318) as claimed in claim 4 wherein at least part of the outer portion (28) is deflected towards the pointed or sharpened part or end (24) of the piercing element (22, 222, 322), and wherein the base (20) slopes downwardly away from where it is connected to the piercing element (22, 222, 322), the slope extending in a direction towards the pointed or sharpened part/end (24) of the piercing element (22, 222, 322), or towards the axis of the piercing element (22, 222, 322).

6. A skin penetration device (302) for administering a test substance to the skin of a recipient, the skin penetration device (302) comprising:
- A first layer (304),
- a second layer (306) disposed on the first layer (304), the second layer (306) comprising at least one protrusion (308) thereby providing a hollow space within the protrusion (308) for containing a substance to be applied to the skin of the recipient, and wherein the first layer (304) comprises an aperture (314) and is arranged such that the protrusion (308) of the second layer (306) is locatable or is located within the aperture (314),
- a third layer (312) disposed on the second layer (306), and
- a skin-piercing component (318) as claimed in any preceding claim, the skin-piercing component (318) being disposed within and/or above the aperture (314) of the first layer (304) and between the second (306) and third layers (312).

7. A skin penetration device (302) as claimed in claim 6 wherein the second layer (306) comprises a plurality of protrusions (308), the first layer (304) comprises a plurality of corresponding apertures (314), and there is a corresponding plurality of skin-piercing components (318).

8. A skin penetration device (302) as claimed in claim 6 or claim 7, wherein the skin-piercing component (318) has a width dimension, and the aperture (314) of the first layer (304) has a width dimension, wherein the width of the aperture (314) is less than the width of the skin-piercing component (318).

9. A skin penetration device (302) as claimed in any one of claims 6 to 8 wherein the third layer (312) has one or more protrusions (332) that are arranged extending over the aperture or apertures (314) of the first layer (304), wherein the skin-piercing component (318) is located within the protrusion (332).

10. A skin penetration device (302) as claimed in any one of claims 6 to 9 wherein the skin-piercing component (318) is adhered to, held by, or integrally formed with the third layer (312), and wherein the third layer (312) conforms to the shape of the skin-piercing component (318) when the skin penetration device (302) is assembled.

11. A skin penetration device (2, 102, 202) for administering a test substance to the skin of a recipient, the skin penetration device (2, 102, 202) comprising:
- a first layer (4, 104, 204),
- a second layer (6, 106, 206) disposed on the first layer (4, 104, 204), the second layer (6, 106, 206) comprising at least one protrusion (8, 108, 208) thereby providing a hollow space within the protrusion (8, 108, 208) and between the first (4, 104, 204) and second layers (6, 106, 206) for containing a substance to be applied to the skin of the recipient,
- a third layer (12, 212) disposed on the second layer (6, 106, 206), the third layer (12, 212) comprising an aperture (14, 214) and being arranged such that the protrusion (8, 108, 208) of the second layer is located within the aperture (14, 214),
- a fourth layer (16, 116, 216) disposed on the third layer (12, 212), covering the aperture (14, 214) of the third layer (12, 212), and
- a skin-piercing component (18, 118, 218, 318) as claimed in any one of claims 1 to 5, the skin-piercing component (18, 118, 218, 318) being disposed within and/or above the aperture (14, 214) of the third layer (12, 212) and between the second (6, 106, 206) and fourth layers (16, 116, 216).

12. A skin penetration device (2, 102, 202) as claimed in claim 11 wherein the second (6, 106, 206) layer comprises a plurality of protrusions (8, 108, 208), the third layer (12, 212) comprises a plurality of corresponding apertures (14, 214), and there is a corresponding plurality of skin-piercing components (18, 118, 218, 318).

13. A skin penetration device (2, 102, 202) as claimed in claim 11 or claim 12 wherein the skin-piercing component (18, 118, 218, 318) is adhered to or integrally formed with the fourth layer (16, 116, 216), and wherein the thickness of the third layer (12, 212) functions to space the fourth layer (16, 116, 216), and therefore the skin-piercing component (18, 118, 218, 318), apart from the protrusion (8, 108, 208) of the second layer (6, 106, 206).

14. A skin-penetration device (2, 102, 202) as claimed in any one of claims 11 to 13 wherein the fourth layer (16, 116, 216) has a protrusion (32, 132) arranged extending over the aperture (14, 214) of the third layer (12, 212), and wherein the skin-piercing component (18, 118, 218, 318) is located within the protrusion (32, 132), wherein the protrusion (32, 132) of the fourth layer (16, 116, 216) is reinforced.

15. A skin-penetration device (2, 102, 202) as claimed in any one of claims 11 to 14 wherein the protrusion (8, 108, 208) of the second layer (6, 106, 206) is configured to receive and guide the skin-piercing component (18, 118, 218, 318) and wherein the protrusion (8, 108, 208) of the second layer (6, 106, 206) has a textured surface (252).

## Patentansprüche

1. Hautdurchdringende Komponente (18, 118, 218, 318) einer Hautpenetrationsvorrichtung, wobei die hautdurchdringende Komponente (18) eine Basis (20) und ein oder mehrere durchdringende Elemente (22, 222, 322) hat, die sich von der Basis (20) erstrecken, und wobei die hautdurchdringende Komponente (18, 118, 218, 318) eine Ruheauslegung hat und dazu ausgelegt ist, derart in Richtung der Ruheauslegung vorgespannt zu werden, dass sie verformt werden kann, doch in die Ruheauslegung zurückkehrt, wenn die Verformungskraft entfernt wird, und **dadurch gekennzeichnet, dass** das eine oder die mehreren durchdringenden Elemente (22, 222, 322) gebildet werden, indem ein Teil der Basis (20) der hautdurchdringenden Komponente (18, 118, 218, 318) ausgestanzt und das ausgestanzte Teil von der Basis (20) weggebogen wird.

2. Hautdurchdringende Komponente (18, 118, 218, 318) nach Anspruch 1, wobei die Basis (20) im Wesentlichen kuppelförmig ist, und wobei sich das durchdringende Element (22, 222, 322) von der Oberseite der Kuppel in eine Richtung an der Unterseite der Kuppel vorbei erstreckend angeordnet ist, und wobei die hautdurchdringende Komponente (18, 118, 218, 318) dazu ausgelegt ist, dem Benutzer taktiles Feedback bereitzustellen, wenn ausreichend Kraft auf die Komponente (18, 118, 218, 318) aufgebracht wurde.

3. Hautdurchdringende Komponente (18, 118, 218, 318) nach Anspruch 2, wobei die Basis (20) dazu ausgelegt ist, zu ermöglichen, dass sich die Basis (20) aus der Kuppelform in der Ruheauslegung in eine abgeflachte oder invertierte Auslegung bewegt, wobei die Basis (20) eine oder mehrere Einschnitte oder Ausschnitte hat, um zu ermöglichen, sie abzuflachen oder zu invertieren, wobei die hautdurchdringende Komponente (18, 118, 218, 318) dazu angeordnet ist, aus der Ruheauslegung in eine abgeflachte oder invertierte Auslegung zu springen, wenn eine ausreichende Kraft aufgebracht wurde, wobei die plötzliche Änderung in der Auslegung, wenn die hautdurchdringende Komponente (18, 118, 218, 318) in Richtung der Haut gedrückt wird, der Person, die die Vorrichtung verabreicht, ein taktiles Feedback bereitstellt und der Person versichert, dass ausreichend Kraft aufgebracht wurde und die hautdurchdringende Komponente (18, 118, 218, 318) die Haut durchdrungen hat.

4. Hautdurchdringende Komponente (18, 118, 218, 318) nach einem vorhergehenden Anspruch, wobei die Basis (20) einen Innenabschnitt (26) und einen Außenabschnitt (28) hat, und wobei der Außenabschnitt (28) aus einer Vielzahl von Flanschen (30) gebildet ist, die sich von dem Innenabschnitt (26) nach außen erstrecken, und wobei das durchdringende Element (22, 222, 322) an dem Innenabschnitt (26) mit der Basis (20) verbunden ist.

5. Hautdurchdringende Komponente (18, 118, 218, 318) nach Anspruch 4, wobei mindestens ein Teil des Außenabschnitts (28) in Richtung des spitzen oder scharfen Teils oder Endes (24) des durchdringenden Elements (22, 222, 322) abgelenkt ist, und wobei die Basis (20) von dort, wo sie mit dem durchdringenden Element (22, 222, 322) verbunden ist, nach unten geneigt ist, wobei sich die Neigung in eine Richtung in Richtung des spitzen oder scharfen Teils/Endes (24) des durchdringenden Elements (22, 222, 322) erstreckt oder in Richtung der Achse des durchdringenden Elements (22, 222, 322).

6. Hautpenetrationsvorrichtung (302) zur Verabreichung einer Testsubstanz an die Haut eines Empfängers, wobei die Hautpenetrationsvorrichtung (302) umfasst:
- eine erste Schicht (304),
- eine zweite Schicht (306), die auf der ersten Schicht (304) angeordnet ist, wobei die zweite Schicht (306) mindestens einen Vorsprung (308) umfasst und dadurch einen Hohlraum in dem Vorsprung (308) zum Aufnehmen einer auf die Haut des Empfängers aufzubringenden Substanz bereitstellt, und wobei die erste Schicht (304) eine Öffnung (314) umfasst und derart angeordnet ist, dass der Vorsprung (308) der zweiten Schicht (306) in der Öffnung (314) positionierbar oder positioniert ist,
- eine dritte Schicht (312), die auf der zweiten Schicht (306) angeordnet ist, und
- eine hautdurchdringende Komponente (318) nach einem vorhergehenden Anspruch, wobei die hautdurchdringende Komponente (318) in und/oder über der Öffnung (314) der ersten Schicht (304) und zwischen der zweiten (306) und der dritten Schicht (312) angeordnet ist.

7. Hautpenetrationsvorrichtung (302) nach Anspruch 6, wobei die zweite Schicht (306) eine Vielzahl von Vorsprüngen (308) umfasst, wobei die erste Schicht (304) eine Vielzahl von entsprechenden Öffnungen (314) umfasst, und es eine entsprechende Vielzahl von hautdurchdringenden Komponenten (318) gibt.

8. Hautpenetrationsvorrichtung (302) nach Anspruch 6 oder Anspruch 7, wobei die hautdurchdringende Komponente (318) eine Breitenabmessung hat und die Öffnung (314) der ersten Schicht (304) eine Breitenabmessung hat, wobei die Breite der Öffnung (314) kleiner Ist als die Breite der hautdurchdringenden Komponente (318).

9. Hautpenetrationsvorrichtung (302) nach einem der Ansprüche 6 bis 8, wobei die dritte Schicht (312) einen oder mehrere Vorsprünge (332) hat, die sich über die Öffnung oder Öffnungen (314) der ersten Schicht (304) erstreckend angeordnet sind, wobei die hautdurchdringende Komponente (318) in dem Vorsprung (332) positioniert ist.

10. Hautpenetrationsvorrichtung (302) nach einem der Ansprüche 6 bis 9, wobei die hautdurchdringende Komponente (318) auf der dritten Schicht (312) haftet, von dieser gehalten wird oder einstückig damit gebildet ist, und wobei die dritte Schicht (312) der Form der hautdurchdringenden Komponente (318) entspricht, wenn die Hautpenetrationsvorrichtung (302) zusammengesetzt ist.

11. Hautpenetrationsvorrichtung (2, 102, 202) zur Verabreichung einer Testsubstanz an die Haut eines Empfängers, wobei die Hautpenetrationsvorrichtung (2, 102, 202) umfasst:
- eine erste Schicht (4, 104, 204),
- eine zweite Schicht (6, 106, 206), die auf der ersten Schicht (4, 104, 204) angeordnet ist, wobei die zweite Schicht (6, 106, 206) mindestens einen Vorsprung (8, 108, 208) umfasst und dadurch einen Hohlraum in dem Vorsprung (8, 108, 208) und zwischen der ersten (4, 104, 204) und der zweiten Schicht (6, 106, 206) zum Aufnehmen einer auf die Haut des Empfängers aufzubringenden Substanz bereitstellt,
- eine dritte Schicht (12, 212), die auf der zweiten Schicht (6, 106, 206) angeordnet ist, wobei die dritte Schicht (12, 212) eine Öffnung (14, 214) umfasst und derart angeordnet ist, dass der Vorsprung (8, 108, 208) der zweiten Schicht in der Öffnung (14, 214) positioniert ist,
- eine vierte Schicht (16, 116, 216), die auf der dritten Schicht (12, 212) angeordnet ist und die Öffnung (14, 214) der dritten Schicht (12, 212) abdeckt, und
- eine hautdurchdringende Komponente (18, 118, 218, 318) nach einem der Ansprüche 1 bis 5, wobei die hautdurchdringende Komponente (18, 118, 218, 318) in und/oder über der Öffnung (14, 214) der dritten Schicht (12, 212) und zwischen der zweiten (6, 106, 206) und der vierten Schicht (16, 116, 216) angeordnet ist.

12. Hautpenetrationsvorrichtung (2, 102, 202) nach Anspruch 11, wobei die zweite (6, 106, 206) Schicht eine Vielzahl von Vorsprüngen (8, 108, 208) umfasst, wobei die dritte Schicht (12, 212) eine Vielzahl von entsprechenden Öffnungen (14, 214) umfasst, und es eine entsprechende Vielzahl von hautdurchdringenden Komponenten (18, 118, 218, 318) gibt.

13. Hautpenetrationsvorrichtung (2, 102, 202) nach Anspruch 11 oder Anspruch 12, wobei die hautdurchdringende Komponente (18, 118, 218, 318) auf der vierten Schicht (16, 116, 216) haftet oder einstückig damit gebildet ist, und wobei die Dicke der dritten Schicht (12, 212) dazu fungiert, die vierte Schicht (16, 116, 216) und damit die hautdurchdringende Komponente (18, 118, 218, 318) von dem Vorsprung (8, 108, 208) der zweiten Schicht (6, 106, 206) zu beabstanden.

14. Hautpenetrationsvorrichtung (2, 102, 202) nach einem der Ansprüche 11 bis 13, wobei die vierte Schicht (16, 116, 216) einen Vorsprung (32, 132) hat, der sich über die Öffnung (14, 214) der dritten Schicht (12, 212) erstreckend angeordnet ist, und wobei die hautdurchdringende Komponente (18, 118, 218, 318) in dem Vorsprung (32, 132) positioniert ist, wobei der Vorsprung (32, 132) der vierten Schicht (16, 116, 216) verstärkt ist.

15. Hautpenetrationsvorrichtung (2, 102, 202) nach einem der Ansprüche 11 bis 14, wobei der Vorsprung (8, 108, 208) der zweiten Schicht (6, 106, 206) dazu ausgelegt ist, die hautdurchdringende Komponente (18, 118, 218, 318) aufzunehmen und zu führen, und wobei der Vorsprung (8, 108, 208) der zweiten Schicht (6, 106, 206) eine strukturierte Oberfläche (252) hat.

## Revendications

1. Composant de perforation cutanée (18, 118, 218, 318) d'un dispositif de pénétration cutanée, le composant de perforation cutanée (18) ayant une base (20) et un ou plusieurs éléments de perforation (22, 222, 322) s'étendant à partir de la base (20), et le composant de perforation cutanée (18, 118, 218, 318) ayant une configuration de repos et étant configuré pour être sollicité vers la configuration de repos de sorte qu'il peut être déformé mais retourne à la configuration de repos lorsque la force de déformation est supprimée, et **caractérisé en ce que** le ou les éléments de perforation (22, 222, 322) sont formés par découpage d'une partie de la base (20) du composant de perforation cutanée (18, 118, 218, 318) et pliage de la partie découpée à l'écart de la base (20).

2. Composant de perforation cutanée (18, 118, 218, 318) selon la revendication 1, la base (20) étant sensiblement en forme de dôme, et l'élément de perforation (22, 222, 322) étant agencé en s'étendant à partir du sommet du dôme dans une direction dépassant le fond du dôme, et le composant de perforation cutanée (18, 118, 218, 318) étant configuré pour fournir une rétroaction tactile à l'utilisateur lorsqu'une force suffisante a été appliquée au composant (18, 118, 218, 318).

3. Composant de perforation cutanée (18, 118, 218, 318) selon la revendication 2, la base (20) étant configurée pour permettre à la base (20) de se déplacer de la forme de dôme dans la configuration de repos à une configuration aplatie ou inversée, ladite base (20) ayant une ou plusieurs incisions ou découpes pour lui permettre d'être aplatie ou inversée, le composant de perforation cutanée (18, 118, 218, 318) étant agencé pour passer de la configuration de repos à une configuration aplatie ou inversée lorsqu'une force suffisante a été appliquée, le changement soudain de configuration lorsque le composant de perforation cutanée (18, 118, 218, 318) est pressé contre la peau fournissant à la personne administrant le dispositif un retour tactile et garantissant à la personne qu'une force suffisante a été appliquée et que le composant de perforation cutanée (18, 118, 218, 318) a percé la peau.

4. Composant de perforation cutanée (18, 118, 218, 318) selon l'une quelconque des revendications précédentes, la base (20) ayant une partie intérieure (26) et une partie extérieure (28), et la partie extérieure (28) étant formée d'une pluralité de rebords (30) s'étendant à partir de la partie intérieure (26), et l'élément de perforation (22, 222, 322) étant relié à la base (20) au niveau de la partie intérieure (26).

5. Composant de perforation cutanée (18, 118, 218, 318) selon la revendication 4, au moins une partie de la partie extérieure (28) étant déviée vers la partie ou extrémité pointue ou aiguisée (24) de l'élément de perforation (22, 222, 322), et la base (20) étant inclinée vers le bas à partir de l'endroit où elle est reliée à l'élément de perforation (22, 222, 322), la pente s'étendant dans une direction vers la partie/extrémité pointue ou aiguisée (24) de l'élément de perforation (22, 222, 322) ou vers l'axe de l'élément de perforation (22, 222, 322).

6. Dispositif de pénétration cutanée (302) destiné à administrer une substance d'essai sur la peau d'un receveur, le dispositif de pénétration cutanée (302) comprenant :
- une première couche (304),
- une deuxième couche (306) disposée sur la première couche (304), la deuxième couche (306) comprenant au moins une protubérance (308), créant ainsi un espace creux dans la protubérance (308) pour contenir une substance à appliquer sur la peau du receveur, et la première couche (304) comprenant une ouverture (314) et étant agencée de telle sorte que la protubérance (308) de la deuxième couche (306) est localisable ou se trouve dans l'ouverture (314),
- ne troisième couche (312) disposée sur la deuxième couche (306), et
- un composant de perforation cutanée (318) selon l'une quelconque des revendications précédentes, le composant de perforation cutanée (318) étant disposé à l'intérieur et/ou au-dessus de l'ouverture (314) de la première couche (304) et entre les deuxième (306) et troisième couches (312).

7. Dispositif de pénétration cutanée (302) selon la revendication 6, la deuxième couche (306) comprenant une pluralité de protubérances (308), la première couche (304) comprenant une pluralité d'ouvertures correspondantes (314), et ayant une pluralité correspondante de composants de perforation cutanée (318).

8. Dispositif de pénétration cutanée (302) selon la revendication 6 ou la revendication 7, le composant de perforation cutanée (318) ayant une dimension en largeur, et l'ouverture (314) de la première couche (304) ayant une dimension en largeur, la largeur de l'ouverture (314) étant inférieure à la largeur du composant de perforation cutanée (318).

9. Dispositif de pénétration cutanée (302) selon l'une quelconque des revendications 6 à 8, la troisième couche (312) comportant une ou plusieurs protubérances (332) qui sont agencées s'étendant sur l'ouverture ou les ouvertures (314) de la première couche (304), le composant de perforation cutanée (318) étant situé à l'intérieur de la protubérance (332).

10. Dispositif de pénétration cutanée (302) selon l'une quelconque des revendications 6 à 9, le composant de perforation cutanée (318) étant collé à, maintenu par ou formé d'un seul tenant avec la troisième couche (312), et la troisième couche (312) épousant la forme du composant de perforation cutanée (318) lorsque le dispositif de pénétration cutanée (302) est assemblé.

11. Dispositif de pénétration cutanée (2, 102, 202) destiné à administrer une substance d'essai sur la peau d'un receveur, le dispositif de pénétration cutanée (2, 102, 202) comprenant :
- une première couche (4, 104, 204),
- une deuxième couche (6, 106, 206) disposée sur la première couche (4, 104, 204), la deuxième couche (6, 106, 206) comprenant au moins une protubérance (8, 108, 208), créant ainsi un espace creux à l'intérieur de la protubérance (8, 108, 208) et entre la première couche (4, 104, 204) et la deuxième couche (6, 106, 206) pour contenir une substance à appliquer sur la peau du receveur,
- une troisième couche (12, 212) disposée sur la deuxième couche (6, 106, 206), la troisième couche (12, 212) comprenant une ouverture (14, 214) et étant disposée de telle sorte que la protubérance (8, 108, 208) de la deuxième couche se trouve à l'intérieur de l'ouverture (14, 214),
- une quatrième couche (16, 116, 216) disposée sur la troisième couche (12, 212), recouvrant l'ouverture (14, 214) de la troisième couche (12, 212), et
- un composant de perforation cutanée (18, 118, 218, 318) selon l'une quelconque des revendications 1 à 5, le composant de perforation cutanée (18, 118, 218, 318) étant disposé à l'intérieur et/ou au-dessus de l'ouverture (14, 214) de la troisième couche (12, 212) et entre la deuxième couche (6, 106, 206) et la quatrième couche (16, 116, 216).

12. Dispositif de pénétration cutanée (2, 102, 202) selon la revendication 11, la deuxième couche (6, 106, 206) comprenant une pluralité de protubérances (8, 108, 208), la troisième couche (12, 212) comprenant une pluralité d'ouvertures correspondantes (14, 214), et ayant une pluralité correspondante de composants de perforation cutanée (18, 118, 218, 318).

13. Dispositif de pénétration cutanée (2, 102, 202) selon la revendication 11 ou la revendication 12, le composant de perforation cutanée (18, 118, 218, 318) adhérant à la quatrième couche (16, 116, 216) ou étant formé d'un seul tenant avec celle-ci, et l'épaisseur de la troisième couche (12, 212) servant à espacer la quatrième couche (16, 116, 216), et donc le composant de perforation cutanée (18, 118, 218, 318), à part de la protubérance (8, 108, 208) de la deuxième couche (6, 106, 206) .

14. Dispositif de pénétration cutanée (2, 102, 202) selon l'une quelconque des revendications 11 à 13, la quatrième couche (16, 116, 216) ayant une protubérance (32, 132) agencée s'étendant sur l'ouverture (14, 214) de la troisième couche (12, 212), et le composant de perforation cutanée (18, 118, 218, 318) étant situé à l'intérieur de la protubérance (32, 132), la protubérance (32, 132) de la quatrième couche (16, 116, 216) étant renforcée.

15. Dispositif de pénétration cutanée (2, 102, 202) selon l'une quelconque des revendications 11 à 14, la protubérance (8, 108, 208) de la deuxième couche (6, 106, 206) étant configurée pour recevoir et guider le composant de perforation cutanée (18, 118, 218, 318), et la protubérance (8, 108, 208) de la deuxième couche (6, 106, 206) ayant une surface texturée (252).
